# EUROPEAN PATENT APPLICATION

(11) **EP 4 253 528 A1**
(43) Date of publication of application: **04.10.2023**
(21) Application number: 21898100.9
(22) Date of filing: 26.11.2021
(51) Int. Cl.: C12N 5/07

(54) **THREE-DIMENSIONAL CELL TISSUE PRODUCTION METHOD AND THREE-DIMENSIONAL CELL TISSUE**

(30) Priority: 26.11.2020 JP 2020196310
(71) Applicant: TOPPAN INC., Tokyo 110-0016 (JP)
(72) Inventor: KITANO Shiro, Tokyo 110-0016 (JP); TSUKAMOTO Kei, Tokyo 110-0016 (JP)
(74) Representative: Plasseraud IP
(86) International application number: PCT/JP2021/043393
(87) International publication number: WO 2022/114128

(57) **Abstract**

Provided are a method of producing a three-dimensional cell tissue, the method including: a step of obtaining a mixture containing cells, a cationic substance, an extracellular matrix component and a polyelectrolyte; a step of gelling the mixture to obtain a gel composition; and a step of incubating the gel composition to obtain a three-dimensional cell tissue, and a three-dimensional cell tissue containing: cells; a cationic substance; an extracellular matrix component; a polyelectrolyte; and a gel component.

## Description

### [Technical Field]

The present invention relates to methods of producing a three-dimensional cell tissue, and three-dimensional cell tissues.

The present application is based on and claims the benefit of priority from Japanese Patent Application No. 2020-196310 filed November 26, 2020, the content of which is incorporated herein by reference.

### [Background Art]

In recent years, the superiority of using three-dimensional cell tissues organized three-dimensionally over cells grown on a flat plate has been shown in the fields of regenerative medicine and assay systems for drugs that require an environment close to that of a living body. Accordingly, various techniques for constructing three-dimensional cell tissues in vitro have been developed.

The inventors of the present invention have previously developed a technique for producing a three-dimensional cell tissue, including: a step of obtaining a mixture containing cells suspended in a solution containing at least a cationic buffer solution, an extracellular matrix component and a polyelectrolyte; a step of collecting the cells from the obtained mixture to form a cell aggregate on a substrate; and a step of culturing the cells to obtain a three-dimensional cell tissue (e.g., see PTL 1). Three-dimensional cell tissues can be used for, for example, biological tissue models or solid cancer models for use in various assays such as drug screening.

### [Citation List]

### [Patent Literature]

PTL 1: JP 6639634 B

### [Summary of the Invention]

### [Technical Problem]

However, in order for the three-dimensional cell tissues to maintain the thickness as manufactured over time, there is room for further improvement.

Therefore, an object of the present invention is to provide a technique for suppressing a decrease in thickness of three-dimensional cell tissue over time.

### [Solution to the Problem]

The present invention includes the following aspects.
[1] A method of producing a three-dimensional cell tissue, the method including: a step of obtaining a mixture containing cells, a cationic substance, an extracellular matrix component and a polyelectrolyte; a step of gelling the mixture to obtain a gel composition; and a step of incubating the gel composition to obtain a three-dimensional cell tissue.
[2] The production method according to [1], wherein the gel composition includes a gel component in which at least one selected from the group consisting of the extracellular matrix component, agarose, pectin and fibrin monomers is gelled.
[3] The production method according to [2], wherein the gel component is a fibrin gel in which fibrin monomers are gelled, and the step of gelling includes a step of mixing thrombin and fibrinogen with the mixture.
[4] The production method according to [3], wherein the step of gelling includes a step of adding the thrombin to the mixture and a step of adding the fibrinogen to the mixture to which the thrombin has been added, whereby the fibrin gel is formed to gel the mixture.
[5] The production method according to any one of [1] to [4], wherein the extracellular matrix component is selected from the group consisting of collagen, laminin, fibronectin, vitronectin, elastin, tenascin, entactin, fibrin, proteoglycan, and combinations thereof.
[6] The production method according to any one of [1] to [5], wherein a total content of the extracellular matrix component in the mixture is 0.005 mg/mL or more and 1.5 mg/mL or less.
[7] The production method according to any one of [1] to [6], wherein the polyelectrolyte is selected from the group consisting of glycosaminoglycan, dextran sulfate, rhamnan sulfate, fucoidan, carrageenan, polystyrene sulfonic acid, polyacrylamide-2-methylpropanesulfonic acid, polyacrylic acid, and combinations thereof.
[8] The production method according to any one of [1] to [7], wherein the polyelectrolyte content in the mixture is 0.005 mg/mL or more.
[9] The production method according to any one of [1] to [8], wherein the step of gelling includes: a step of applying an external force to the mixture to obtain a cell aggregate containing the cells, the cationic substance, the extracellular matrix component and the polyelectrolyte; and a step of gelling, as the mixture, the cell aggregate to obtain the gel composition.
[10] A three-dimensional cell tissue containing: cells; a cationic substance; an extracellular matrix component; a polyelectrolyte; and a gel component.
[11] A three-dimensional cell tissue containing: cells; a cationic substance; a polyelectrolyte; and a gel component.
[12] The three-dimensional cell tissue according to [11], wherein the gel component is a gel component in which at least one selected from the group consisting of a first extracellular matrix component, agarose, pectin and fibrin monomers is gelled.
[13] The three-dimensional cell tissue according to [11], further containing a second extracellular matrix component, wherein the gel component is a gel component in which at least one selected from the group consisting of agarose, pectin and fibrin monomers is gelled.
[14] The three-dimensional cell tissue according to any one of [10] to [13], wherein a maximum thickness of a slice obtained by cutting the three-dimensional cell tissue on day 8 of production along a line passing through the center of gravity in a direction perpendicular to the upper surface of the three-dimensional cell tissue is 80% or more of a maximum thickness of a slice obtained by cutting the three-dimensional cell tissue immediately after production along a line passing through the center of gravity in a direction perpendicular to the upper surface of the three-dimensional cell tissue.

### [Advantageous Effects of the Invention]

According to the present invention, a technique for suppressing a decrease in thickness of three-dimensional cell tissue over time can be provided.

### [Brief Description of the Drawings]

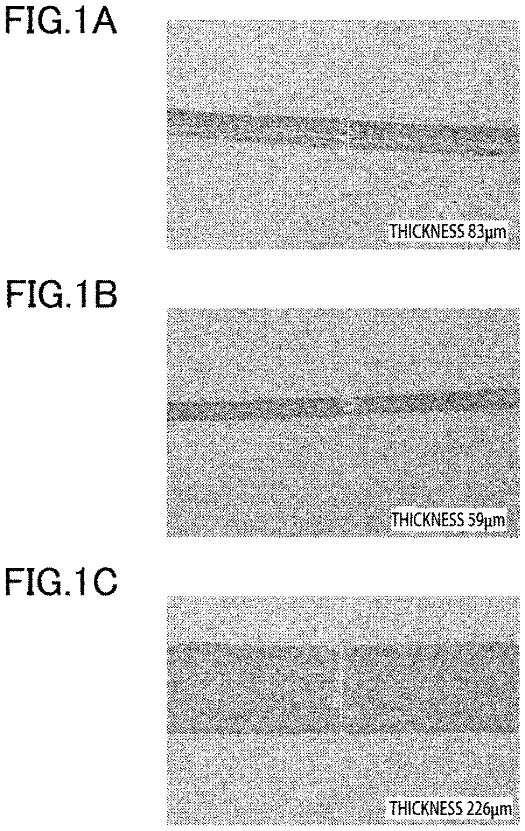

Fig. 1A is a representative micrograph of a thin slice of a three-dimensional cell tissue measured in Experimental Example 1.
Fig. 1B is a representative micrograph of a thin slice of a three-dimensional cell tissue measured in Experimental Example 1.
Fig. 1C is a representative micrograph of a thin slice of a three-dimensional cell tissue measured in Experimental Example 1.

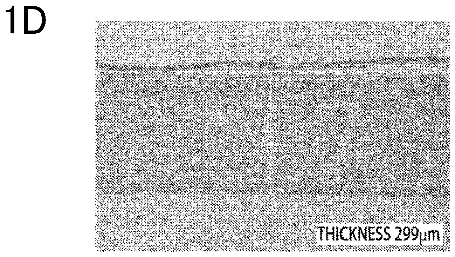

Fig. 1D is a representative micrograph of a thin slice of a three-dimensional cell tissue measured in Experimental Example 1.

### [Description of the Embodiments]

### [Method of Producing Three-Dimensional Cell Tissue]

In an embodiment, the present invention is a method of producing a three-dimensional cell tissue, the method including: a step of obtaining a mixture containing cells, a cationic substance, an extracellular matrix component and a polyelectrolyte; a step of gelling the mixture to obtain a gel composition; and a step of incubating the gel composition to obtain a three-dimensional cell tissue.

According to the production method of the present embodiment, a decrease in thickness of three-dimensional cell tissue over time can be suppressed. Specifically, as will be described later in the examples, a maximum thickness of a slice obtained by cutting the three-dimensional cell tissue on day 8 of production along the line passing through the center of gravity in a direction perpendicular to the upper surface thereof can be maintained at 80% or more of a maximum thickness of a slice obtained by cutting the three-dimensional cell tissue immediately after production along the line passing through the center of gravity in a direction perpendicular to the upper surface thereof. The maximum thickness of the three-dimensional cell tissue on day 8 of production can also be referred to as the maximum thickness of the slice of the three-dimensional cell tissue obtained by cutting the three-dimensional cell tissue on day 8 of incubation of the gel composition along the line passing through the center of gravity in a direction perpendicular to the upper surface thereof. Further, the maximum thickness of the three-dimensional cell tissue immediately after production can also be referred to as the maximum thickness of the slice of the three-dimensional cell tissue obtained by cutting the three-dimensional cell tissue immediately after incubation of the gel composition along the line passing through the center of gravity in a direction perpendicular to the upper surface thereof. The term "immediately after production of the three-dimensional cell tissue" as used herein may refer to 5 minutes to 72 hours, preferably 1 day (24 hours), after the start of incubation of the gel composition. Further, "day 8 of production" may refer to 8 days (preferably 192 hours) after the start of incubation of the gel composition.

As will be described later in the examples, according to the production method of the present embodiment, it is possible to produce three-dimensional cell tissues having a thickness which has been difficult to produce in the conventional art.

The thickness of the slice obtained by cutting the three-dimensional cell tissue along the line passing through the center of gravity in a direction perpendicular to the upper surface thereof refers to the thickness of the slice taken at substantially the center part of the three-dimensional cell tissue. The shape of the three-dimensional cell tissue depends on the vessel used to produce the three-dimensional cell tissue, and may be a cylindrical shape, for example, when the three-dimensional cell tissue is produced using a cylindrical cell culture insert. In this case, the shape of the three-dimensional cell tissue as viewed from the upper side is circular, and the center of gravity as viewed from the upper side is the center of the circle. The shape of the three-dimensional cell tissue is not limited to a cylindrical shape, and can be any shape according to the purpose. Specifically, for example, a polygonal prism shape such as a triangular prism shape or a rectangular prism shape can be exemplified.

Specifically, the thickness of the slice obtained by cutting the three-dimensional cell tissue along the line passing through the center of gravity in a direction perpendicular to the upper surface thereof is measured as follows. Each three-dimensional cell tissue is fixed by subjecting the cultured cell aggregate to a formalin buffer solution (e.g., product number "062-01661", FUJIFILM Wako Pure Chemical Corporation). After the three-dimensional cell tissue is embedded in paraffin, a thin slice taken along the line passing through the center of gravity in a direction perpendicular to the upper surface of the three-dimensional cell tissue (upper surface of the cell culture insert when the three-dimensional cell tissue is produced using the cell culture insert) is produced. Subsequently, the thin slice is subjected to hematoxylin-eosin (also referred to as HE) stain and observed with a microscope to measure a maximum thickness of the three-dimensional cell tissue.

The term "three-dimensional cell tissue" as used herein refers to a three-dimensional cell aggregate. The uses of three-dimensional cell tissues include, but are not limited to, biological tissue models and solid cancer models. Examples of the biological tissue models include skin, hair, bone, cartilage, tooth, cornea, blood vessel, lymphatic vessel, heart, liver, pancreas, nerve and esophagus models. Examples of the solid cancer models include gastric cancer, esophageal cancer, colorectal cancer, colon cancer, rectal cancer, pancreatic cancer, breast cancer, ovarian cancer, prostate cancer, renal cell cancer and liver cancer models.

Further, the form of the three-dimensional cell tissue is not particularly limited, and may be, for example, a three-dimensional cell tissue formed by culturing cells in a vessel such as a cell culture insert, a three-dimensional cell tissue formed by culturing cells in a scaffold of a natural biopolymer such as collagen or a synthetic polymer, a cell aggregate (also referred to as a spheroid), or a sheet-like cell structure.

The method of producing a three-dimensional cell tissue according to the present embodiment includes: a step (a) of obtaining a mixture containing cells, a cationic substance, an extracellular matrix component and a polyelectrolyte; a step (b) of gelling the mixture to obtain a gel composition; and a step (c) of incubating the gel composition to obtain a three-dimensional cell tissue. Each step will be described below.

First, in step (a), a mixture is obtained by mixing cells, a cationic substance, an extracellular matrix component and a polyelectrolyte. The cells, the cationic substance, the extracellular matrix component and the polyelectrolyte may be mixed in an aqueous solvent. Examples of the aqueous solvent include, but are not limited to, water, buffer solutions and media.

### (Cells)

In the production method of the present embodiment, the cells are not particularly limited, and cells derived from mammals such as humans, monkeys, dogs, cats, rabbits, pigs, cows, mice and rats can be used. Also, the site of origin of the cells is not particularly limited, and the cells may be somatic cells, such as those derived from bone, muscle, viscera, nerve, brain, skin and blood, or may be reproductive cells, or cancer cells.

Examples of the somatic cells derived from blood include immune cells such as lymphocytes, neutrophils, macrophages and dendritic cells. Examples of the cancer cells include the cells of gastric cancer, esophageal cancer, colorectal cancer, colon cancer, rectal cancer, pancreatic cancer, breast cancer, ovarian cancer, prostate cancer, renal cell cancer and liver cancer.

Moreover, the cells may be pluripotent stem cells such as induced pluripotent stem cells (iPS cells) and embryonic stem cells (ES cells), or may be tissue stem cells.

The cells may be cultured cells such as primary cells, subcultured cells and cell line cells. These cells may be used singly or in combination of two or more.

### (Cationic Substance)

As the cationic substance, any substance having a positive charge can be used as long as it does not adversely affect the growth of cells and formation of cell aggregate described later. Examples of the cationic substance include, but are not limited to, cationic buffers, such as tris-hydrochloric acid, tris-maleic acid, bis-tris and HEPES, ethanolamine, diethanolamine, triethanolamine, polyvinylamine, polyallylamine, polylysine, polyhistidine, polyarginine, and the like. In particular, a cationic buffer is preferred, and tris-hydrochloric acid buffer is more preferred.

The concentration of the cationic substance in the mixture in step (a) is not particularly limited as long as it does not adversely affect the growth of cells and formation of cell aggregate. The concentration of the cationic substance used in the present embodiment is preferably 10 to 100 mM relative to the total volume of the mixture, and may be, for example, 20 to 90 mM, 30 to 80 mM, 40 to 70 mM, or 45 to 60 mM.

When a cationic buffer is used as the cationic substance, the pH of the cationic buffer solution is not particularly limited as long as it does not adversely affect the growth of cells and formation of cell aggregate. The pH of the cationic buffer solution used in the present embodiment is preferably 6.0 to 8.0. For example, the pH of the cationic buffer solution used in the present embodiment may be 7.0, 7.1, 7.2, 7.3, 7.4, 7.5, 7.6, 7.7, 7.8, 7.9 or 8.0. The pH of the cationic buffer solution used in the present embodiment is more preferably 7.2 to 7.6, and still more preferably approximately 7.4.

### (Extracellular Matrix Component)

As the extracellular matrix component, any component constituting an extracellular matrix (also referred to as ECM) can be used as long as it does not adversely affect the growth of cells and formation of cell aggregate described later. Examples of the extracellular matrix component include, but are not limited to, collagen, laminin, fibronectin, vitronectin, elastin, tenascin, entactin, fibrin, proteoglycan, and combinations thereof. The extracellular matrix component may also be modified forms or variants of the above components. These extracellular matrix components may be used singly or in combination of two or more.

Examples of the proteoglycan include chondroitin sulfate proteoglycan, heparan sulfate proteoglycan, keratan sulfate proteoglycan and dermatan sulfate proteoglycan. In particular, collagen, laminin and fibronectin are preferred, and collagen is particularly preferred as the extracellular matrix component.

The total content of the extracellular matrix component in the mixture in step (a) is not particularly limited as long as it does not adversely affect the growth of cells and formation of cell aggregate. The total content of the extracellular matrix component relative to the total volume of the mixture may be 0.005 mg/mL or more and 1.5 mg/mL or less, 0.005 mg/mL or more and 1.0 mg/mL or less, 0.01 mg/mL or more and 1.0 mg/mL or less, 0.025 mg/mL or more and 1.0 mg/mL or less, or 0.025 mg/mL or more and 0.1 mg/mL or less. The extracellular matrix component may be dissolved in an appropriate solvent before use. Examples of the solvent include, but are not limited to, water, buffer solutions and acetic acid. In particular, buffer solutions or acetic acid is preferred.

### (Polyelectrolyte)

The term "polyelectrolyte" as used herein refers to a polymer having a dissociable functional group in the polymer chain. As the polyelectrolyte used in the present embodiment, any polyelectrolyte can be used as long as it does not adversely affect the growth of cells and formation of cell aggregate. Examples of the polyelectrolyte include, but are not limited to, glycosaminoglycans such as heparin, chondroitin sulfate (e.g., chondroitin 4-sulfate, or chondroitin 6-sulfate), heparan sulfate, dermatan sulfate, keratan sulfate and hyaluronic acid; dextran sulfate, rhamnan sulfate, fucoidan, carrageenan, polystyrene sulfonic acid, polyacrylamide-2-methylpropanesulfonic acid, polyacrylic acid, and combinations thereof. The polyelectrolyte may be derivatives of those described above. These polyelectrolytes may be used singly or in combination of two or more.

The polyelectrolyte is preferably a glycosaminoglycan. In particular, heparin, chondroitin sulfate and dermatan sulfate are preferred, and heparin is particularly preferred.

The concentration of the polyelectrolyte in the mixture in step (a) is not particularly limited as long as it does not adversely affect the growth of cells and formation of cell aggregate. Unlike the extracellular matrix component, the polyelectrolyte is effective at any concentration as long as it is below the solubility limit, and does not inhibit the effects of the extracellular matrix component. The concentration of polyelectrolyte relative to the total volume of the mixture is preferably 0.005 mg/mL or more, and may be 0.005 mg/mL or more and 1.0 mg/mL or less, 0.01 mg/mL or more and 1.0 mg/mL or less, 0.025 mg/mL or more and 1.0 mg/mL or less, or 0.025 mg/mL or more and 0.1 mg/mL or less.

The polyelectrolyte may be dissolved in an appropriate solvent before use. Examples of the solvent include, but are not limited to, water and buffer solutions. When a cationic buffer solution is used as the above cationic substance, the polyelectrolyte may be dissolved in a cationic buffer solution before use.

A mixing ratio (final concentration ratio) between the polyelectrolyte and the extracellular matrix component in the mixture in step (a) is preferably 1:2 to 2:1, and may be 1:1.5 to 1.5:1, or may be 1:1.

In step (a), the cells, the cationic substance, the extracellular matrix component and the polyelectrolyte can be mixed in a suitable vessel such as a dish, tube, flask, bottle, well plate or cell culture insert. The mixing may be performed in a vessel used in step (b).

Further, the mixture in step (a) may also contain other components than the cells, the cationic substance, the extracellular matrix component and the polyelectrolyte. Examples of the other components include gelling agents, cell culture media, and the like necessary for obtaining a gel composition in step (b).

### (Gelling Agent)

Examples of the gelling agent include extracellular matrix components; agarose; pectin; combinations of fibrinogen and thrombin. The gelling agent may be contained in advance in the mixture in step (a), or may be added, in step (b) described below, to the mixture obtained in step (a).

In step (b) subsequent to step (a), the mixture obtained in step (a) is gelled to obtain a gel composition. The method of gelling depends on the gelling agent used, and, for example, the mixture obtained in step (a) may be subjected to gelling conditions. Alternatively, a gelling agent may be added to the mixture obtained in step (a), and then the mixture may be subjected to gelling conditions.

For example, when an extracellular matrix component is gelled to obtain a gel composition, the gelling conditions include, for example, allowing the mixture obtained in step (a) to stand at about 37°C. Further, an additive such as a calcium salt may be added to the mixture obtained in step (a). As a result, the extracellular matrix component contained in the mixture obtained in step (a) is gelled, whereby a gel composition is obtained. Alternatively, an extracellular matrix component may be added to the mixture obtained in step (a), and then the mixture may be gelled by allowing it to stand at about 37°C.

Further, when agarose is used as the gelling agent, agarose may be added to the mixture obtained in step (a), and, after dissolving the agarose under temperature conditions higher than or equal to the melting point of the agarose used, the mixture may be gelled by allowing it to stand under temperature conditions lower than or equal to the freezing point of the agarose used. For example, the temperature at which agarose is dissolved may be 50°C to 90°C. Further, the temperature at which the dissolved agarose is gelled may be 37°C to 40°C. The addition ratio of the agarose to the mixture may be 0.5% to 4% relative to the total volume of the mixture.

Further, when pectin is used as the gelling agent, pectin may be added to the mixture obtained in step (a). As a result, the pectin is gelled by divalent ions such as calcium ions contained in the mixture, whereby a gel composition is obtained. The addition ratio of the pectin to the mixture may be 0.3 to 1.0% relative to the total volume of the mixture.

Further, fibrinogen and thrombin may be used as the gelling agent. Thrombin, a type of serine protease, cleaves the fibrinogen to form fibrin monomers. The fibrin monomers polymerize with each other by the action of calcium ions to form poorly-soluble fibrin polymers. The fibrin polymers are cross-linked in vivo by the action of factor XIII (fibrin stabilizing factor) to form mesh-like fibers called stabilized fibrin, causing blood coagulation. In the present specification, a gel composition which is gelled due to formation of fibrin polymers may also be referred to as a fibrin gel. In the present specification, polymerization of fibrin monomers to form a fibrin polymer is described as gelation of fibrin monomers.

That is, the step (b) of obtaining a gel composition may include a step of mixing thrombin and fibrinogen with the mixture obtained in step (a). The gel composition in step (b) may contain a fibrin gel as a gel component.

In this case, the step (b) of obtaining a gel composition preferably include a step (b1) of adding thrombin to the mixture obtained in step (a), and a step (b2) of adding fibrinogen to the mixture to which thrombin has been added, whereby the fibrin gel is formed to gel the mixture.

When fibrinogen and thrombin are used as the gelling agent, the fibrinogen concentration in the mixture in step (b) may be 0.5 mg/mL or more and 70 mg/mL or less, preferably 0.5 mg/mL or more and 45 mg/mL or less, and more preferably 0.5 mg/mL or more and 25 mg/mL or less. When the concentration is 0.5 mg/mL or more and 70 mg/mL or less, the fibrin monomers are easily gelled by being mixed with thrombin. The concentration of 25 mg/mL or less facilitates dissolving in the mixture. Further, thrombin is preferably dissolved or dispersed in the mixture in step (b).

The thrombin concentration in the mixture in step (b) is preferably 1.0 mg/mL or more, and more preferably 5.0 mg/mL or more. When the thrombin concentration in the mixture is 1.0 mg/mL or more, the fibrin monomers are easily gelled. The upper limit of the thrombin concentration in the mixture is not particularly limited, but may be 60 mg/mL. The upper limit and the lower limit of the thrombin concentration in the mixture can be combined as appropriate. For example, the thrombin concentration in the mixture may be 1.0 mg/mL or more and 60 mg/mL or less, or may be 5.0 mg/mL or more and 60 mg/mL or less.

The thrombin concentration and the fibrinogen concentration in the mixture in step (b) are preferably determined depending on the respective concentrations. For example, when the fibrinogen concentration in the mixture is 0.5 mg/mL or more and 5.0 mg/mL or less, the thrombin concentration is preferably 1.0 mg/mL or more and 60 mg/mL or less. Further, when the fibrinogen concentration in the mixture is 10 mg/mL or more and 70 mg/mL or less, the thrombin concentration is preferably 5 mg/mL or more and 60 mg/mL or less.

If the mixture contains a substance with anticoagulant properties (typically, when heparin is selected as the polyelectrolyte), it is conceivable that fibrin may not be polymerized. For this reason, a substance with anticoagulant properties is not usually used together with fibrin. In the present embodiment, however, polymerization of fibrin is not inhibited, although it is not clear whether this is due to the combination of materials or the concentration being low.

In addition, the inventors have found that adding fibrinogen first to the mixture obtained in step (a) may cause the mixture to be gelled depending on the conditions. It is presumed that the fibrinogen is cleaved by some protease contained in the mixture obtained in step (a), and forms fibrin monomers. Therefore, in mixing thrombin and fibrinogen with the mixture obtained in step (a), it is preferred to mix thrombin first and then mix fibrinogen with the mixture. Alternatively, a gel composition can be obtained by adding only fibrinogen as a gelling agent in step (b).

Thus, the gel composition formed in step (b) may contain a gel component in which at least one of the extracellular matrix component, agarose, pectin and fibrin monomers is gelled.

Subsequently, in step (c), the gel composition obtained in step (b) is incubated to obtain a three-dimensional cell tissue. The time required for culturing the gel composition to obtain a three-dimensional cell tissue may be 5 minutes to 192 hours, or may be 12 hours to 144 hours, or may be 24 hours to 72 hours. Step (c) promotes adhesion between cells contained in the gel composition, whereby the effect of stabilizing the three-dimensional cell tissue is achieved.

In step (c), cell culture can be performed under culture conditions suitable for the cells to be cultured. Those skilled in the art can select an appropriate medium according to the cell type and desired function. Examples of the medium include, but are not limited to, media such as DMEM, EMEM, MEMα, RPMI-1640, McCoy's 5A and Ham's F-12, and media obtained by adding about 1 to 20 vol% of serum to these media. Examples of serum include bovine serum (CS), fetal bovine serum (FBS), fetal horse serum (HBS), and the like. Conditions such as the temperature and atmospheric composition of the culture environment may also be adjusted according to the cells to be cultured.

The vessel used in step (c) may be the same as the vessel used in step (b). In step (c), the vessel used in step (b) may be used as it is, or another vessel may be used.

Upon cell culture, a substance for suppressing deformation of the obtained three-dimensional cell tissue (such as tissue contraction, peeling at the tissue edge, or the like) may be added to the medium. Examples of such a substance include, but are not limited to, Y-27632, which is a Rho-associated coiled-coil forming kinase/Rho binding kinase (ROCK) inhibitor.

Step (c) may be performed after step (a) and step (b) are performed two or more times. By repeating step (a) and step (b), a three-dimensional cell tissue having a plurality of layers can be produced. That is, a three-dimensional cell tissue having an increased thickness can be produced.

Further, step (a) and step (b) may be repeated using a different cell population for each repetition to form a three-dimensional cell tissue composed of different types of cells. For example, after the first step (a) and step (b) are performed, the second step (a) may be performed using a cell population different from that in the first step (a). Then, by performing the second step (b), a layer containing the cell population used in the second step (a) can be formed on the layer containing the cell population used in the first step (a). By repeating step (a) and step (b) a plurality of times as described above, a three-dimensional cell tissue composed of different types of cell population can be produced.

### (Cell Aggregate)

In the production method of the present embodiment, the step (b) of gelling may include: a step (a') of applying an external force to the mixture obtained in step (a) to obtain a cell aggregate containing the cells, the cationic substance, the extracellular matrix component and the polyelectrolyte; and a step of gelling, as the mixture obtained in step (a), the cell aggregate obtained in step (a'), to obtain the gel composition.

That is, a method of producing a three-dimensional cell tissue of the present embodiment includes: a step (a) of obtaining a mixture containing cells, a cationic substance, an extracellular matrix component and a polyelectrolyte; a step (a') of applying an external force to the mixture to obtain a cell aggregate containing the cells, the cationic substance, the extracellular matrix component and the polyelectrolyte; a step (b') of gelling the cell aggregate to obtain a gel composition; and a step (c) of incubating the gel composition to obtain a three-dimensional cell tissue.

The term "cell aggregate" as used herein refers to a structure composed of cells combined into a single unit. The cell aggregate includes cell precipitates obtained by centrifugation, filtration, or the like. In an embodiment, the cell aggregate is a slurry-like viscous body. The term "slurry-like viscous body" refers to a gel-like cell aggregate as described in Akihiro Nishiguchi et al., Cell-cell crosslinking by bio-molecular recognition of heparin-based layer-by-layer nanofilms, Macromol Biosci., 15 (3), 312-317, 2015.

The cell aggregate may be formed by allowing the mixture obtained in step (a) to stand in an appropriate vessel, or may be formed by placing the mixture obtained in step (a) in an appropriate vessel and subjecting it to, for example, centrifugation, magnetic separation, filtration, or the like to aggregate the cells. That is, applying an external force in step (a') may refer to allowing the mixture to stand to apply gravity, or performing centrifugation, magnetic separation, filtration, or the like. After the cells are aggregated by standing, centrifugation, magnetic separation, filtration, or the like, the liquid part may be removed or may not be removed.

The vessel used in step (a') may be a culture vessel used for cell culture. The culture vessel may be a vessel having a material and a shape typically used for cell or microorganism culture. Examples of the material of the culture vessel include, but are not limited to, glass, stainless steel, plastic, and the like. Examples of the culture vessel include, but are not limited to, a dish, tube, flask, bottle, well plate, cell culture insert, and the like. Preferably, the vessel is, at least in part, made of a material that allows liquid to pass through without allowing cells in the liquid to pass through. Examples of such a vessel include, but are not limited to, cell culture inserts, such as Transwell (registered trademark) insert, Netwell (registered trademark) insert, Falcon (registered trademark) cell culture insert, and Millicell (registered trademark) cell culture insert.

The conditions of centrifugation are not particularly limited as long as they do not adversely affect the growth of cells. For example, the cells can be aggregated to obtain a cell aggregate by placing the mixture in a cell culture insert and centrifuging it at 10°C at 400 × g for 1 minute.

Step (b') of gelling the cell aggregate to obtain a gel composition can be performed in the same manner as step (b) of gelling the mixture obtained in step (a) to obtain a gel composition described above.

For example, when an extracellular matrix component is used as the gelling agent, the cell aggregate obtained in step (a') may be allowed to stand at about 37°C, or an extracellular matrix component may be added to the cell aggregate obtained in step (a') and then the cell aggregate may be gelled by allowing it to stand at about 37°C. Further, an additive such as a calcium salt may be added to the mixture obtained in step (a').

Further, when agarose is used as the gelling agent, agarose may be added to the cell aggregate obtained in step (a'), and, after dissolving the agarose under temperature conditions higher than or equal to the melting point of the agarose used, the cell aggregate may be gelled by allowing it to stand under temperature conditions lower than or equal to the freezing point of the agarose used. For example, the temperature at which agarose is dissolved may be 50°C to 90°C. Further, the temperature at which the dissolved agarose is gelled may be 37°C to 40°C.

Further, when pectin is used as the gelling agent, pectin may be added to the cell aggregate obtained in step (a'). As a result, the pectin is gelled by divalent ions such as calcium ions contained in the mixture, whereby a gel composition is obtained.

Further, fibrinogen and thrombin may be used as the gelling agent. That is, the step (b') of obtaining a gel composition may include a step of mixing thrombin and fibrinogen with the cell aggregate obtained in step (a'). The gel composition in step (b') may contain a fibrin gel as a gel component.

In this case, the step (b') of obtaining a gel composition preferably include a step (b1') of adding thrombin to the cell aggregate obtained in step (a'), and a step (b2') of adding fibrinogen to the cell aggregate to which thrombin has been added, whereby the fibrin gel is formed to gel the mixture.

Subsequently, in step (c), the gel composition obtained in step (b') is incubated to obtain a three-dimensional cell tissue. Step (c) is the same as that described above.

### [Three-Dimensional Cell Tissue]

In one embodiment, the present invention provides a three-dimensional cell tissue containing: cells; a cationic substance; an extracellular matrix component; a polyelectrolyte; and a gel component. In another aspect, the present invention provides a three-dimensional cell tissue containing: cells; a cationic substance; a polyelectrolyte; and a gel component. According to the three-dimensional cell tissue of the present embodiment, a decrease in thickness over time is suppressed.

According to the three-dimensional cell tissue of the present embodiment, a maximum thickness of a slice obtained by cutting the three-dimensional cell tissue on day 8 of production along the line passing through the center of gravity in a direction perpendicular to the upper surface thereof is preferably 80% or more, 90% or more, or 95% or more, or may be 100% or more of a maximum thickness of a slice obtained by cutting the three-dimensional cell tissue immediately after production along the line passing through the center of gravity in a direction perpendicular to the upper surface thereof. As used herein, the "day 8 of production" and "immediately after production" are the same as those described above.

In the three-dimensional cell tissue of the present embodiment, the cells, the cationic substance, the extracellular matrix component, the polyelectrolyte and the gel component are the same as those described above.

The gel component is a gel component in which at least one of the extracellular matrix component, agarose, pectin and fibrin monomers is gelled.

When the gel component is a gelled extracellular matrix, another extracellular matrix may not be necessarily contained. Specifically, the three-dimensional cell tissue may contain cells, a cationic substance, a polyelectrolyte and a gel component. Further, when the gel component is a gel component in which at least one of agarose, pectin and fibrin monomers is gelled, the three-dimensional cell tissue may contain cells, an extracellular matrix component, a cationic substance, a polyelectrolyte and a gel component.

When the gel component is a gelled agarose, the gel component content in the three-dimensional cell tissue may be 0.5% to 4% relative to the total volume of the three-dimensional cell tissue.

When the gel component is a gelled pectin, the gel component content in the three-dimensional cell tissue may be 0.3 to 1.0 relative to the total volume of the three-dimensional cell tissue.

When the gel component is gelled fibrin monomers, the gel component content in the three-dimensional cell tissue may be 0.5 mg/mL or more and 70 mg/mL or less, preferably 0.5 mg/mL or more and 45 mg/mL or less, and more preferably 0.5 mg/mL or more and 25 mg/mL or less relative to the total volume of the three-dimensional cell tissue.

The present invention includes other aspects as follows.
[1] A method of producing a three-dimensional cell tissue, the method including: a step of obtaining a mixture containing cells, a cationic substance, an extracellular matrix component and a polyelectrolyte; a step of mixing at least one of thrombin and fibrinogen with the mixture to obtain a gel composition; and a step of incubating the gel composition to obtain a three-dimensional cell tissue.
[2] The method of producing a three-dimensional cell tissue according to [1], wherein the step of gelling is a step of mixing the fibrinogen with the mixture to obtain a gel composition.
[3] The production method according to [1], wherein the step of gelling includes: a step of adding the thrombin to the mixture; and a step of adding the fibrinogen to the mixture to which the thrombin has been added.
[4] The production method according to any one of [1] to [3], wherein the fibrinogen content in the mixture is 0.5 mg/mL or more and 60 mg/mL or less.
[5] The production method according to any one of [1] to [4], wherein the thrombin content in the mixture is 1.0 mg/mL or more and 40 mg/mL or less.
[6] The production method according to any one of [1] to [5], wherein the extracellular matrix component is at least one extracellular matrix selected from the group consisting of collagen, laminin and fibronectin.
[7] The production method according to any one of [1] to [6], wherein the extracellular matrix component is collagen.
[8] The production method according to any one of [1] to [7], wherein a total content of the extracellular matrix component in the mixture is 0.005 mg/mL or more and 1.5 mg/mL or less.
[9] The production method according to any one of [1] to [8], wherein the polyelectrolyte is glycosaminoglycan.
[10] The production method according to [9], wherein the polyelectrolyte is at least one glycosaminoglycan selected from the group consisting of heparin, chondroitin sulfate and dermatan sulfate.
[11] The production method according to any one of [1] to [10], wherein the polyelectrolyte content in the mixture is 0.005 mg/mL or more.
[12] The production method according to any one of [1] to [11], wherein the step of gelling includes: a step of applying an external force to the mixture to obtain a cell aggregate containing the cells, the cationic substance, the extracellular matrix component and the polyelectrolyte; and a step of gelling, as the mixture, the cell aggregate to obtain the gel composition.
[13] A three-dimensional cell tissue including: cells; a cationic substance; an extracellular matrix component; polyelectrolyte; and gelled fibrin monomers.
[14] The three-dimensional cell tissue according to [13], wherein the gelled fibrin monomer content in the three-dimensional cell tissue is 0.5 mg/mL or more and 60 mg/mL or less.
[15] The three-dimensional cell tissue according to [13] or [14], wherein a maximum thickness of a slice obtained by cutting the three-dimensional cell tissue on day 8 of production along a line passing through the center of gravity in a direction perpendicular to the upper surface of the three-dimensional cell tissue is 80% or more of a maximum thickness of a slice obtained by cutting the three-dimensional cell tissue immediately after production along a line passing through the center of gravity in a direction perpendicular to the upper surface of the three-dimensional cell tissue.

### Examples

The present invention will be described in more detail using the examples, but the present invention is not limited to the following examples.

### [Experimental Example 1]

### (Production of Three-Dimensional Cell Tissues)

Three-dimensional cell tissues containing no fibrin gel and three-dimensional cell tissues containing a fibrin gel were respectively produced. Neonatal human dermal fibroblasts NHDF (product number "CC-2509", Lonza) were uses as the cells.

### < Production of Three-Dimensional Cell Tissues Containing No Fibrin Gel>>

A DMEM medium (product number "043-30085", FUJIFILM Wako Pure Chemical Corporation) containing 10% fetal bovine serum (FBS) and 1% antibiotic solution (penicillin, streptomycin) was prepared (hereinafter, also referred to as a "general-purpose culture medium"). Further, a medium was prepared by mixing a general-purpose culture medium and a vascular cell growth medium (product name "EGM-2MV", product number "CC-3202", Lonza) at 1:1 (volume ratio) (hereinafter, also referred to as a "dedicated culture medium").

1×10⁶, 2×10⁶, 3×10⁶, 4×10⁶ and 8×10⁶ NHDF cells were each suspended in a 50 mM tris-HCl buffer solution (pH 7.4) containing 0.05 mg/mL heparin (product number "H3149-100KU", Sigma) and 0.05 mg/mL collagen (product number "ASC-1-100-100", Sigma).

Then, each cell suspension was centrifuged at room temperature at 1,000 × g (gravitational acceleration) for 1 minute, and, after removing the supernatant, resuspended in an appropriate amount of the dedicated culture medium. Subsequently, each cell suspension was seeded in a 24-well cell culture insert (product number "3470", Corning Incorporated).

Then, the cell culture insert was centrifuged at room temperature at 400 × g for 1 minute to obtain cell aggregates. Subsequently, an appropriate amount of the dedicated culture medium was added to the cell culture insert, and the mixture was cultured in a CO₂ incubator (37°C, 5% CO₂) for 8 days. During the culture, the medium was exchanged as appropriate.

### < Production of Three-Dimensional Cell Tissues Containing Fibrin Gel>>

A thrombin solution was prepared by dissolving thrombin (product number "T4648-10KU", Sigma) in the general-purpose culture medium described above to produce final concentration of 10 Unit/mL.

A fibrinogen solution was prepared by dissolving fibrinogen (product number "F8630-5G", Sigma) in the DMEM medium to produce final concentration of 10 mg/mL. Further, a medium was prepared by mixing the fibrinogen solution and the general-purpose culture medium at 1:1 (volume ratio) (hereinafter, also referred to as a "fibrinogen-added medium").

Then, 1×10⁶, 2×10⁶, 3×10⁶, 4×10⁶, 8×10⁶ NHDF cells were each suspended in a 50 mM tris-HCl buffer solution (pH 7.4) containing 0.05 mg/mL heparin (product number "H3149-100KU", Sigma) and 0.05mg/mL collagen (product number "ASC-1-100-100", Sigma).

Then, each cell suspension was centrifuged at room temperature at 1,000 × g for 1 minute, and, after removing the supernatant, resuspended in the thrombin solution. Subsequently, the thrombin solution in which the cells were dissolved and the fibrinogen-added culture were mixed at 1:2 (volume ratio), and 100 µL of the mixed solution was seeded in a 24-well cell culture insert (product number "3470", Corning Incorporated).

Then, the cell culture insert was allowed to stand in a CO₂ incubator (37°C, 5% CO₂) until the mixture was gelled. Subsequently, an appropriate amount of the dedicated culture medium was added to the cell culture insert, and the mixture was cultured in a CO₂ incubator (37°C, 5% CO₂) for 8 days. During the culture, the medium was exchanged as appropriate.

### <<Tissue Section Analysis>>

On day 1 and day 8 of culture of cell aggregate, each three-dimensional cell tissue was fixed using a formalin buffer solution (product number "062-01661", FUJIFILM Wako Pure Chemical Corporation). Then, each three-dimensional cell tissue was removed from the cell culture insert, embedded in paraffin, and cut along the line passing through the center of gravity in a direction perpendicular to the upper surface of the three-dimensional cell tissue (in other words, upper surface of the cell culture insert) to prepare a thin slice. Subsequently, the thin slice was subjected to hematoxylin-eosin (also referred to as HE) stain and observed with a microscope to measure a maximum thickness of the three-dimensional cell tissue.

Figs. 1A to 1D are representative micrographs of a thin slice of each three-dimensional cell tissue. Fig. 1A is a micrograph of a tissue slice on day 1 of culture of a three-dimensional cell tissue containing no fibrin gel (produced 2×10⁶ cell counts). Fig. 1B is a micrograph of a tissue slice on day 8 of culture of a three-dimensional cell tissue containing no fibrin gel (produced 2×10⁶ cell counts). Fig. 1C is a micrograph of a tissue slice on day 1 of culture of a three-dimensional cell tissue containing a fibrin gel (produced 2×10⁶ cell counts). Fig. 1D is a micrograph of a tissue slice on day 8 of culture of a three-dimensional cell tissue containing a fibrin gel (produced 2×10⁶ cell counts).

Table 1 below shows the results of measuring a maximum thickness of the respective three-dimensional cell tissues containing no fibrin gel. Table 2 shows the results of measuring a maximum thickness of the respective three-dimensional cell tissues containing a fibrin gel. In Tables 1 and 2, "thickness on day 1" indicates the maximum thickness (µm) of a slice obtained by cutting the three-dimensional cell tissue on day 1 of culture along the line passing through the center of gravity in a direction perpendicular to the upper surface thereof, and "thickness on day 8" indicates the maximum thickness (µm) of a slice obtained by cutting the three-dimensional cell tissue on day 8 of culture along the line passing through the center of gravity in a direction perpendicular to the upper surface thereof. Further, "preparation failed" indicates that the cells were detached from the cell culture insert, resulting in a failure of three-dimensional cell tissue preparation, and "measurement failed" indicates that measurement could not be performed.

In Tables 1 and 2, "thickness retention ratio" is a value calculated by the following formula (1).

Thickness retention ratio (%) = maximum thickness of a slice obtained by cutting the three-dimensional cell tissue on day 8 of culture along the line passing through the center of gravity in a direction perpendicular to the upper surface thereof / maximum thickness of a slice obtained by cutting the three-dimensional cell tissue on day 1 of culture along the line passing through the center of gravity in a direction perpendicular to the upper surface thereof × 100 ...... (1)

In Tables 1 and 2, "retainability" is a result of evaluation according to the following evaluation criteria.

### (Evaluation Criteria)

Good: Thickness retention ratio calculated by the above formula (1) is 80% or more
Poor: Thickness retention ratio calculated by the above formula (1) is less than 80%

**[Table 1]**

| Three-dimensional cell tissue containing no fibrin gel | | | | | |
|---|---|---|---|---|---|
| Cell count | 1 × 10⁶ | 2 × 10⁶ | 3 × 10⁶ | 4 × 10⁶ | 8×10⁶ |
| Thickness (µm) on day 1 | 60 | 83 | 132 | 179 | Preparation failed |
| | | | | | Measurement failed |
| Thickness (µm) on day 8 | 34 | 59 | 95 | 113 | Preparation failed |
| | | | | | Measurement failed |
| Thickness retention ratio (%) | 57 | 71 | 72 | 63 | Measurement failed |
| Retainability | Poor | Poor | Poor | Poor | Poor |

**[Table 2]**

| Three-dimensional cell tissue containing fibrin gel | | | | | |
|---|---|---|---|---|---|
| Cell count | 1 × 10⁶ | 2 × 10⁶ | 3 × 10⁶ | 4 × 10⁶ | 8 × 10⁶ |
| Thickness (µm) on day 1 | 201 | 226 | 269 | 370 | 445 |
| Thickness (µm) on day 8 | 199 | 299 | 307 | 488 | 627 |
| Thickness retention ratio (%) | 99 | 132 | 114 | 132 | 141 |
| Retainability | Good | Good | Good | Good | Good |

As seen from the results, in the three-dimensional cell tissues containing a fibrin gel, a thickness retention ratio of 80% or more was achieved under any cell count condition, suppressing a decrease in thickness of the three-dimensional cell tissue over time.

On the other hand, in the three-dimensional cell tissues containing no fibrin gel, a three-dimensional cell tissue could not be prepared under the condition of using 8 × 10⁶ cells. In addition, under other cell count conditions, the thickness retention ratio was less than 80%, and a decrease in thickness of the three-dimensional cell tissue over time was found.

Furthermore, the three-dimensional cell tissues containing a fibrin gel were found to form thicker tissues compared with the three-dimensional cell tissues containing no fibrin gel.

### [Experimental Example 2]

### (Three-Dimensional Cell Tissue Preparation 1)

Three-dimensional cell tissues containing a fibrin gel were produced. Neonatal human dermal fibroblasts NHDF (product number "CC-2509", Lonza) and human umbilical vein endothelial cells (GFP-HUVEC) (product number: cAP-0001GFP, manufactured by Funakoshi Co., Ltd.) were uses as the cells.

Thrombin solutions were prepared by dissolving thrombin (product number "T4648-10KU", Sigma) in the general-purpose culture medium described above to produce respective final concentrations of 0.5, 1, 5, 10, 20 and 40 Unit/mL.

Fibrinogen solutions were prepared by dissolving fibrinogen (product number "F8630-5G", Sigma) in the DMEM medium to produce respective final concentrations of 0.5, 1, 5, 10, 30 and 60 mg/mL. Further, a medium was prepared by mixing the fibrinogen solution and the general-purpose culture medium at 1:1 (volume ratio) (hereinafter, also referred to as a "fibrinogen-added medium").

Then, 2×10⁶ NHDF cells and 4×10⁵ GFP-HUVEC were suspended in a 50 mM tris-HCl buffer solution (pH 7.4) containing 0.005 mg/mL heparin (product number "H3149-100KU", Sigma) and 0.05 mg/mL collagen (product number "ASC-1-100-100", Sigma).

Then, each cell suspension was centrifuged at room temperature at 1,000 × g for 1 minute, and, after removing the supernatant, resuspended in the thrombin solution. Subsequently, the thrombin solution in which the cells were dissolved and the fibrinogen-added culture were mixed at 1:2 (volume ratio), and 100 µL of the mixed solution was seeded in a 24-well cell culture insert (product number "3470", Corning Incorporated).

Then, the cell culture insert was allowed to stand in a CO₂ incubator (37°C, 5% CO₂) until the mixture was gelled. Subsequently, an appropriate amount of the dedicated culture medium described above was added to the cell culture insert, and the mixture was cultured in a CO₂ incubator (37°C, 5% CO₂) for 8 days. During the culture, the medium was exchanged as appropriate.

### <<Tissue Section Analysis>>

On day 1 and day 8 of culture of cell aggregate, each three-dimensional cell tissue was fixed using a formalin buffer solution (product number "062-01661", FUJIFILM Wako Pure Chemical Corporation). Then, each three-dimensional cell tissue was removed from the cell culture insert, embedded in paraffin, and cut along the line passing through the center of gravity in a direction perpendicular to the upper surface of the three-dimensional cell tissue (in other words, upper surface of the cell culture insert) to prepare a thin slice. Subsequently, the thin slice was subjected to hematoxylin-eosin (HE) stain and observed with a microscope to measure a maximum thickness of the three-dimensional cell tissue.

Table 3 below shows the evaluation results of "retainability" for each three-dimensional cell tissue evaluated according to the same evaluation criteria as in Experimental Example 1.

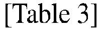

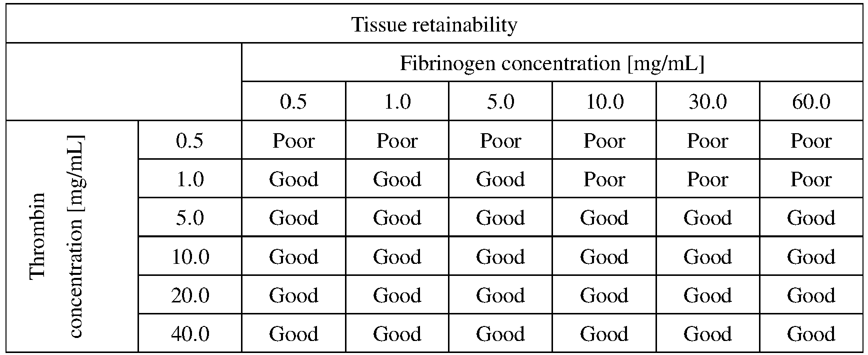

As seen from the results, when the final concentration of thrombin was 5 mg/mL or more, a thickness retention ratio of the three-dimensional cell tissue of 80% or more was achieved in any three-dimensional cell tissue produced under the condition where the final concentration of fibrinogen was 0.5 mg/mL or more, and a decrease in thickness of the three-dimensional cell tissue over time was suppressed.

When the final concentration of thrombin was 1 mg/mL, the thickness retention ratio was found to be 80% or more under the condition where the final concentration of fibrinogen was in a range of 0.5 to 5 mg/mL, and a decrease in thickness of the three-dimensional cell tissue over time was suppressed.

### [Experimental Example 3]

### (Three-Dimensional Cell Tissue Preparation 2)

Three-dimensional cell tissues in which an extracellular matrix was gelled were produced. Neonatal human dermal fibroblasts NHDF (product number "CC-2509", Lonza) and human umbilical vein endothelial cells (GFP-HUVEC) (product number: cAP-0001GFP, manufactured by Funakoshi Co., Ltd.) were uses as the cells.

### < Production of Three-Dimensional Cell Tissues Containing No Gelled Extracellular Matrix>>

1×10⁶ NHDF cells and 2×10⁵ GFP-HUVEC were suspended in a 50 mM tris-HCl buffer solution (pH 7.4) containing 0.05 mg/mL heparin (product number "H3149-100KU", Sigma). Similarly, 2×10⁶ NHDF cells and 4×10⁵ GFP-HUVEC were suspended in the 50 mM tris-HCl buffer solution described above.

Then, each cell suspension was centrifuged at room temperature at 1,000 × g (gravitational acceleration) for 1 minute, and, after removing the supernatant, resuspended in an appropriate amount of the dedicated culture medium described above. Subsequently, each cell suspension was seeded in a 24-well cell culture insert (product number "3470", Corning Incorporated).

Then, the cell culture insert was centrifuged at room temperature at 400 × g for 1 minute to obtain cell aggregates. Subsequently, an appropriate amount of the dedicated culture medium described above was added to the cell culture insert, and the mixture was cultured in a CO₂ incubator (37°C, 5% CO₂) for 8 days. During the culture, the medium was exchanged as appropriate.

### < Production of Three-Dimensional Cell Tissues Containing Gelled Extracellular Matrix>>

1×10⁶ NHDF cells and 2×10⁵ GFP-HUVEC were suspended in a 50 mM tris-HCl buffer solution (pH 7.4) containing 0.05 mg/mL heparin (product number "H3149-100KU", Sigma). Similarly, 2×10⁶ NHDF cells and 4×10⁵ GFP-HUVEC were suspended in a 50 mM tris-HCl buffer solution containing 0.05 mg/mL heparin (product number "H3149-100KU", Sigma).

Subsequently, each cell suspension was centrifuged at room temperature at 1,000 × g for 1 minute, and, after removing the supernatant, mixed with collagen (Cellmatrix Type I-A, Nitta Gelatin Inc.) prepared according to a predetermined procedure specified by the manufacturer, and 100 µL of the mixed solution was seeded in a 24-well cell culture insert (product number "3470", Corning Incorporated).

Then, the cell culture insert was allowed to stand in a CO₂ incubator (37°C, 5% CO₂) until the mixed solution was gelled. Subsequently, an appropriate amount of the dedicated culture medium described above was added to the cell culture insert, and the mixture was cultured in a CO₂ incubator (37°C, 5% CO₂) for 8 days. During the culture, the medium was exchanged as appropriate.

### <<Tissue Section Analysis>>

On day 1 and day 8 of culture of cell aggregate, each three-dimensional cell tissue was fixed using a formalin buffer solution (product number "062-01661", FUJIFILM Wako Pure Chemical Corporation). Then, each three-dimensional cell tissue was removed from the cell culture insert, embedded in paraffin, and cut along the line passing through the center of gravity in a direction perpendicular to the upper surface of the three-dimensional cell tissue (in other words, upper surface of the cell culture insert) to prepare a thin slice. Subsequently, the thin slice was subjected to hematoxylin-eosin (HE) stain and observed with a microscope to measure a maximum thickness of the three-dimensional cell tissue.

Table 4 below shows the results measuring a maximum thickness of the respective three-dimensional cell tissues containing a gelled extracellular matrix and the three-dimensional cell tissues containing a gelled extracellular matrix, the thickness retention ratio, and the evaluation results of "retainability" for each three-dimensional cell tissue evaluated according to the same evaluation criteria as in Experimental Example 1.

**[Table 4]**

| | Three-Dimensional Cell Tissue Containing No Gelled Extracellular Matrix | | Three-Dimensional Cell Tissue Containing Gelled Extracellular Matrix | |
|---|---|---|---|---|
| Cell count | 1 × 10⁶ | 2 × 10⁶ | 1 × 10⁶ | 2 × 10⁶ |
| NHDF Thickness (µm) on day 1 | 57 | 74 | 210 | 279 |
| Thickness (µm) on day 8 | 28 | 51 | 200 | 292 |
| Thickness retention ratio (%) | 49 | 68 | 95 | 104 |
| Retainability | Poor | Poor | Good | Good |

As seen from the results, in the three-dimensional cell tissues containing a gelled extracellular matrix, a thickness retention ratio of 80% or more was achieved under any cell count condition, suppressing a decrease in thickness of the three-dimensional cell tissue over time.

On the other hand, in the three-dimensional cell tissues containing no gelled extracellular matrix, the thickness retention ratio was less than 80% under any cell count condition, and a decrease in thickness of the three-dimensional cell tissue over time was found.

Furthermore, the three-dimensional cell tissues containing a gelled extracellular matrix were found to form thicker tissues compared with the three-dimensional cell tissues containing no gelled extracellular matrix.

### [Industrial Applicability]

According to the present invention, a technique for suppressing a decrease in thickness of three-dimensional cell tissue over time can be provided.

## Claims

1. A method of producing a three-dimensional cell tissue, the method comprising:
a step of obtaining a mixture containing cells, a cationic substance, an extracellular matrix component and a polyelectrolyte;
a step of gelling the mixture to obtain a gel composition; and
a step of incubating the gel composition to obtain a three-dimensional cell tissue.

2. The production method according to claim 1, wherein
the gel composition includes a gel component in which at least one selected from the group consisting of the extracellular matrix component, agarose, pectin and fibrin monomers is gelled.

3. The production method according to claim 2, wherein
the gel component is a fibrin gel in which fibrin monomers are gelled, and
the step of gelling includes a step of mixing thrombin and fibrinogen with the mixture.

4. The production method according to claim 3, wherein
the step of gelling includes:
a step of adding the thrombin to the mixture; and;
a step of adding the fibrinogen to the mixture to which the thrombin has been added, whereby the fibrin gel is formed to gel the mixture.

5. The production method according to any one of claims 1 to 4, wherein
the extracellular matrix component is selected from the group consisting of collagen, laminin, fibronectin, vitronectin, elastin, tenascin, entactin, fibrin, proteoglycan, and combinations thereof.

6. The production method according to any one of claims 1 to 5, wherein
a total content of the extracellular matrix component in the mixture is 0.005 mg/mL or more and 1.5 mg/mL or less.

7. The production method according to any one of claims 1 to 6, wherein
the polyelectrolyte is selected from the group consisting of glycosaminoglycan, dextran sulfate, rhamnan sulfate, fucoidan, carrageenan, polystyrene sulfonic acid, polyacrylamide-2-methylpropanesulfonic acid, polyacrylic acid, and combinations thereof.

8. The production method according to any one of claims 1 to 7, wherein
the polyelectrolyte content in the mixture is 0.005 mg/mL or more.

9. The production method according to any one of claims 1 to 8, wherein
the step of gelling includes:
a step of applying an external force to the mixture to obtain a cell aggregate containing the cells, the cationic substance, the extracellular matrix component and the polyelectrolyte; and
a step of gelling, as the mixture, the cell aggregate to obtain the gel composition.

10. A three-dimensional cell tissue containing:
cells;
a cationic substance;
an extracellular matrix component;
a polyelectrolyte; and
a gel component.

11. A three-dimensional cell tissue containing:
cells;
a cationic substance;
a polyelectrolyte; and
a gel component.

12. The three-dimensional cell tissue according to claim 11, wherein
the gel component is a gel component in which at least one selected from the group consisting of a first extracellular matrix component, agarose, pectin and fibrin monomers is gelled.

13. The three-dimensional cell tissue according to [11], further containing
a second extracellular matrix component, wherein
the gel component is a gel component in which at least one selected from the group consisting of agarose, pectin and fibrin monomers is gelled.

14. The three-dimensional cell tissue according to any one of claims 10 to 13, wherein
a maximum thickness of a slice obtained by cutting the three-dimensional cell tissue on day 8 of production along a line passing through the center of gravity in a direction perpendicular to an upper surface of the three-dimensional cell tissue is 80% or more of a maximum thickness of a slice obtained by cutting the three-dimensional cell tissue immediately after production along a line passing through the center of gravity in a direction perpendicular to the upper surface of the three-dimensional cell tissue.
